# EUROPEAN PATENT SPECIFICATION

(11) **EP 1 464 946 B1**
(45) Date of publication and mention of the grant of the patent: **20.09.2006**
(21) Application number: 03007765.5
(22) Date of filing: 04.04.2003
(51) Int. Cl.: G01N 3/08, G01N 33/36, G01B 5/02

(54) **A testing apparatus and a method for the determination of staple fiber length, shrinkage and crimp properties**
Prüfgerät und Verfahren zur Bestimmung der Länge, Schrumpfung und Kräuseleigengenschaften von Stapelfasern
Appareil de test et méthode pour déterminer la longueur, le rétrécissement et les propriétés de frisure de fibres discontinues

(43) Date of publication of application: 06.10.2004
(73) Proprietor: Saudi Basic Industries Corporation, Riyadh 11422 (SA)
(72) Inventor: Waleed, Al-Lafi, 11551 Riyadh (SA)
(74) Representative: Winkler, Andreas Fritz Ernst

(56) References cited:
- DE-A- 3 017 924
- GB-A- 2 179 741
- US-A- 4 856 342
- US-A- 5 799 103
- "STANDARD TEST METHOF FOR SHRINKAGE OF TEXTILE FIBERS (SINGLE-FIBER TEST)", ANNUAL BOOK OF ASTM STANDARDS, D5104-96, January 1997 (1997-01-00), pages 692-695, XP009015623
- "TOW CRIMP ANALYZER" RESEARCH DISCLOSURE, KENNETH MASON PUBLICATIONS, HAMPSHIRE, GB, no. 124, 1 August 1974 (1974-08-01), pages 13-14, XP002023920 ISSN: 0374-4353

## Description

The present invention relates to a testing apparatus for the determination of the shrinkage, the length and crimp properties such as crimp number, crimp removal and crimp stability of individual staple fibers comprising an upper fixing board adapted to hold at least an upper edge of at least one staple fiber, and at least one clip adapted to be reversibly placed on a lower opposite edge of the staple fiber.

In US 4,856,342 a device for measuring the adhesion of fibers in fiber-reinforced synthetic materials is disclosed. This device includes a clamp which represents a fiber chucking device which consists of two clamping jaws one of which consists of a polish metal plate whereas the second jaw consists partially of a transparent synthetic material plate. The fiber to be tested according to US 4,856,342, is, thus, clamped at one of its end and embedded in a polymer matrix at the other end. With the apparatus of US 4,856,342 the fiber is drawn out of the synthetic material matrix and the associated force-distance diagram is registered.

GB 2 179 741 A is concerned with a measuring frame for textile goods and comprises two planar transparent polymer sheets between which a garment can be placed in order to be no longer restricted in use to, for example, tea-shirts or similar tubular nit devices of equivalent shape with which tubular fabric garments with arms, sleaves, legs or leg-opening in a variety of shapes, sizes and materials can be tested for agreement with manufacturing specification or for agreement with washing and handling dimensional-change performance specification. Non-transparent lengthy jaws are used to be placed on the ham of the forward and real faces of the garment.

US 5,799,103 refers to a method for the automatic characterization of mechanical and/or geometric properties of staple fiber samples as well as a suitable apparatus therefore which makes use of a magazine containing a plurality of staple fibers. This apparatus encompasses an upper clamping jaw which when in a close position transfers the staple fiber sample from the transfer position in the magazine into a measuring position away from the magazine by means of a transporter. After the characterization of the mechanical and/or geometric properties of the staple fiber sample has been completed the upper clamping jaw will open and the staple fiber sample will be removed from the apparatus. For example, the number of crimp argues per unit length of the staple fiber sample is determined with said apparatus by creating an image of predetermined length and predetermined width of the staple fiber sample by means of an imager which is preferably movable in the direction of the longitudinal fiber axis, creating from the image a digital grid whose pixels are deposited in the form of numerical values in a memory whereby said numerical values represent measurements of the lightness at the respective locus of the image and determining from the digital grid number of crimp axe in the depicted staple fiber sample by means of digital image processing. The imager is illuminated by a light source so that the staple fiber sample is situated at the intersection between the light source and the imager. Preferably the light source is a mirror which is irradiated by an optical wave guide and which illuminates the imager indirectly thereby in during in particularly thermal stress on the staple fiber sample.

A prominent feature of a staple fiber is its shrinkage behaviour. For quite a while there exist standard procedures as ASTM Standard D5104-96, a standard test method for shrinkage of textile fibers (single-fiber test), in order to be able to provide comparative data on fiber shrinkage. According to the protocol of D5104-96 a test specimen is clamped to an upper board with its upper edge while to the lower edge of the fiber a clip is attached. Usually clips of known mass made of magnetic alloy which are suitable for extending the test fibers by applying a light load are employed. In general, copper clips have proven to be worthwhile.

Also, another method for the determination of shrinkage of staple fibers is outlined in an information disclosure of Zweigle Textilprüfmaschinen GmbH & Co. KG. According to Zweigle a fiber is shrunk in hot air of defined temperature whereby the shrinkage is measured with a fiber shrinkage tester comprising grip weights, a drying oven, forceps and a velvet pad of contrasting colour. The grip weight being attached to the fiber is inserted with one leg in a holder underneath the clamp of the fiber shrinkage device. In this manner it is ensured that during exposure to hot air the fiber is not subjected to any stretching forces.

Irrespective of which known protocol is applied the test results are always not very precise and error-prone, usually yielding unacceptable standard deviations.

In order to obtain comparative data sets on the shrinkage behaviour of individual staple fibers, D5104-96 even recommends the purchaser and the supplier of staple fibers to conduct separate measurements until the standard deviation for all specimen tested differ no more than the agreed to value. Especially, if there is a statistical bias between the data of the purchaser and the that of the supplier it is recommended to seek competent statistical assistance.

As a matter of fact, present testing methods for the shrinkage of textile fibers are very time consuming, require analytical experts to be involved and are therefore rather inefficient from an economical point of view. This lack of reliability is especially of disadvantage if the purchaser of staple fibers detects a shipment of said staple fibers not to be in agreement with the desired specification. At present, it is also rather time consuming to measure or determine a comprehensive set of staple fiber properties such as length, shrinkage, crimp number, crimp stability and crimp removal.

It is therefore an object of the present invention to provide a testing apparatus for the determination of the shrinkage of single staple fibers which does not exhibit the drawbacks of the state of the art devices and which allows for accurate and reliable shrinkage measurements which does not afford too much time and can also be handled by persons who are not analytical experts, and which furnishes a comprehensive set of data at one time.

These and other objects are accomplished by a testing apparatus according to claim 1.

Provisions may be made that the testing apparatus also comprises at least one heater. The heater being an integral element of the testing apparatus helps to reduce the testing time as well as variations in analysis.

In a preferred embodiment the testing apparatus also comprises a, in particular velvet, pad of contrasting color with regard to the staple fiber and being behind the staple fibre, in particular having a black surface. In order to alleviate the length measurements also a scaling can be applied on the pad besides or behind the staple fibers.

According to another aspect of the present invention the testing apparatus can also comprise a fixing means on or adjacent to the upper fixing board adapted to temporarily hold the clip and/or the lower edge of the staple fiber. If according to a shrinkage test to be conducted it is required to not apply a load to the staple fibers when subjected to heating, it has been found to be most useful to have a fixing means, for example, a hook or a clamp or the like on or adjacent to the fixing board. However, such a fixing means can also be placed a bit further apart from the fixing board as long as no force is applied to the staple fiber during the heating. With the term upper fixing board it should just be pointed at the fact that with this fixing device the upper edges of the fibers to be examined are fixed or clamped.

By illuminating the transparent clip and/or the transparent upper fixing board length measurements can be conducted accurately and also very quickly. Especially by use of a digital monitor which is linked to a data processing equipment a multitude of test results can be gathered and analyzed without any delay. As soon as a desired data set has been collected the standard deviation can be calculated rendering an impression on the accuracy of the measurement.

Provisions may be made that the clip and/or the upper fixing board is/are made from a transparent or translucent polymer material, in particular from polymethylmethacrylate (PMMA) and/or polycarbonate (PC) or transparent or translucent mixtures comprising polymethylmethacrylate and/or polycarbonate.

In another embodiment it is preferred that the upper fixing board is adapted to magnetically clamp or hold at least the upper edge of the staple fiber to be tested.

Further provisions may be made that the testing apparatus of the invention further comprises a weight adapted to be, in particular reversibly, attachable to the clip. If, for example, the weight of the clip as such does not suffice to extend the shrunk clip to its full length a proper measurement can be best conducted by applying a weight to said clip. Therefore such clips are usually designed in such a way that weights can be attached thereto.

Further objects of the present invention have been solved by a method according to claim 8.

Provisions may be made that in addition to the length measurement the shrinkage and/or crimp properties, in particular the crimp number, the crimp removal and the crimp stability of the shrunk fiber are determined and the result recorded in step g).

In a preferred embodiment of the method of the invention a grip weight is, in particular reversibly, attached to the clip at least during the exposure to the testing environment.

Provisions may be made that the testing environment comprises hot air, in particular from about 180° to about 195°C, or hot water, in particular from about 95° to about 99° C, in particular for about 15 minutes.

It is particularly helpful if the recorded data is transferred from the digital monitor to a computer.

Provisions may be made that the length measurements on fibers are made in standard atmosphere, in particular at 20° to 22°C and at 63 to 67 % relative humidity.

It has surprisingly been found that by using a transparent or translucent clip instead of, for example, a copper clip the accuracy and reliability of shrinkage measurements of individual staple fibers can be dramatically increased. Apart from length and shrinkage it is now also possible to also measure crimp properties such as crimp number, crimp removal and crimp stability. Further, for the first time it is possible to provide length, shrinkage and crimp data which can be directly processed by a computer or a data processing equipment thereby furnishing a complete evaluation of a sample of staple fibers which have been subjected to a shrinkage testing. Also, by illuminating the transparent clips with a light source allows for a clear picture of the test samples, especially when mounted on a black surface. Furthermore, the accuracy can even be improved by using a transparent or translucent upper fixing board in addition to transparent or translucent clips thereby eliminating any ambiguities with regard to the position where the clamping of the upper edge of the staple fiber takes place. It is therefore possible to measure very precisely the length, the shrinkage and crimp properties such as crimp number, crimp stability and crimp removal of a staple fiber between two clamping positions, even together at one time.

With the testing apparatus according to the invention it is also possible to determine the crimp properties of staple fibers rather precisely, even the number and location of crimps can be measured, in particular if the clip is detached from the lower edge of the staple fiber after the shrinkage procedure. In general, not only the accuracy can be greatly increased with the testing apparatus of the invention, but also the testing time can be reduced to a significant extent.

### BRIEF DESCRIPTION OF THE DRAWINGS

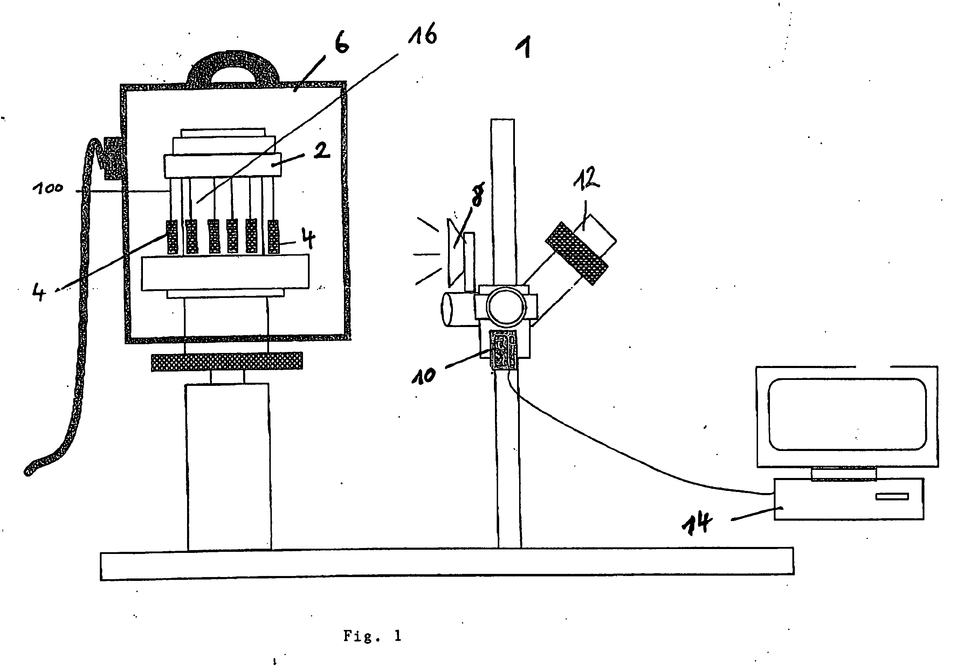

A more complete appreciation of the invention and any of the attended advantages thereof will be readily understood by reference to the following detailed description when considered in connection with the accompanying drawing, wherein the figure depicts a schematic view of a testing apparatus according to the invention including recording and data processing equipment.

### DETAILED DESCRIPTION OF THE INVENTION

In the accompanying drawing a testing apparatus 1 is shown which comprises an upper fixing board 2 to which individual staple fiber samples 100 are attached with their upper ends. These upper ends can either be clamped, for example by a movable bar as described in D5104-96, if necessary with the aid of a high-temperature resistant tape, or be fixed with other known procedures. It is advantageous if the location where the staple fiber is fixed or clamped to the upper board can be determined rather precisely. Said location is preferably rather confined to a defined area or line. Preferably, the upper fixing board is transparent, e.g. made from polymethylmethacrylate (PMMA). To the lower edge of each fiber sample 100 a transparent grip or clip 4 is attached having a minimal load, i.e. which allows the length of the fiber sample to be accurately measured without stretching the sample. The upper fixing board 2 can be easily placed within a heater 6 which is well suited to initiate the shrinkage of the fiber samples. In front of the attached fiber samples a lamp 8 and a digital monitor 10 is installed to record the fiber length of the test samples prior to and after the shrinkage. In order to increase the accuracy of the measurement a microscope 12 is used as well. The data recorded via the digital monitor 10 can be transferred to a computer 14 to store and analyze the collected data.

A typical determination of the shrinkage behaviour of a single staple fiber by use of the testing apparatus according to the invention comprises fixing the individual staple fibers to the upper board 2 of the testing device 1, attaching a transparent grip 4 to the opposite lower edge of the fiber sample 100, attaching the grip 4 to or adjacent to the upper board 2 while the fiber samples are placed in the heater 6 which has been pre-heated to a defined temperature. After a predetermined conditioning time following the heating procedure the fibers 100 are allowed to extend to their full length in front of the black surface 16 by subjecting the fiber samples to the weight of the transparent grip 4. The length of the fiber sample is then measured by aid of a digital monitor 10 and a microscope 12. The length of the staple fiber is also determined prior to the shrinkage procedure in the same manner as described for the shrunk staple fibre. By illuminating the transparent grip 4 the length of the fiber sample can be very precisely determined by measuring the distance between the location of the fiber sample where it is fixed to the upper fixing board 2 and the nip of the transparent grip 4 where it is fixed to said fiber sample. It is particularly advantageous to be able to accurately measure the length not only of one single fiber sample but of a batch of individual fiber samples, e.g. six or even more, in one run.

For comparative studies several series of shrinkage measurements have been conducted at 190°C for 20 min using a conventional shrinkage tester as for example from Zweigle Textilprüfmaschinen GmbH & Co. KG on the one hand, and a shrinkage tester according to the invention on the other hand. In each series the length of the staple fiber has been measured ten times prior to and also ten times after having been subjected to heating under standard conditions as outlined in ASTM standard D5104-96. Whereas with the conventional shrinkage tester according to Zweigle et al. the standard deviation of the percentage of shrinkage has been determined to be 2.48, 3.51 and 1.52 when the average shrinkage behaviour has been calculated to be 10.03 %, 11.35 % and 9.90 %, respectively, with the modified shrinkage test according to the invention standard deviations of 1.35, 0.43 and 0.91 have been obtained for the average percentage of shrinkage of 9.60 %, 10.01 % and 9.90 %, respectively. These results clearly prove the testing apparatus of the invention to be well in advance of conventional testing devices, the test results not only being more accurate but also having been obtained much quicker.

Obviously, numerous modifications and variations of the present invention are possible in the light of the above teaching. It is therefore to be understood that within the scope of the attended claims, the invention may be practiced otherwise than as specifically described herein.

## Claims

1. A testing apparatus (1) for the determination of the shrinkage, the length and crimp properties of individual staple fibers comprising an upper fixing board (2) adapted to hold at least an upper edge of at least one staple fiber (100), at least one clip (4) adapted to be reversibly placed on a lower, opposite edge of the staple fiber (100), **characterized in that** the upper fixing board (2) and the at least one clip (4) are transparent or translucent and **in that** the testing apparatus comprises at least one lamp (8) adapted to illuminate the upper fixing board (2), the clip or clips (4) and the staple fibers (100) when fixed to the upper fixing board (2), at least one microscope (12) and at least one digital monitor (10), both in combination being adapted to measure, to record the fiber length and to transfer the recorded data onto a microprocessor or computer (14).

2. The testing apparatus (1) according to claim 1, further comprising at least one heater (6) for initiating the shrinkage of the staple fibers.

3. The testing apparatus (1) according to claim 1 or 2, further comprising a, in particular velvet, pad (16) of contrasting color with regard to the staple fiber (100) in particular having a black surface, and being behind the staple fiber.

4. The testing apparatus (1) according to one of the preceding claims, further comprising a fixing means on or adjacent to the upper fixing board (2) adapted to temporarily hold the clip (4) and/or the lower edge of the staple fiber (100).

5. The testing apparatus (1) according to one of the preceding claims, wherein the clip (4) and/or the upper fixing board (2) is/are made from a transparent or translucent polymeric material, in particular from polymethylmethacrylate (PMMA) and/or polycarbonate (PC) or transparent or translucent mixtures comprising polymethylmethacrylate and/or polycarbonate.

6. The testing apparatus (1) according to one of the preceding claims, wherein the upper fixing board (2) is adapted to magnetically clamp or hold at least the upper edge of the staple fiber (100) to be tested.

7. The testing apparatus (1) according to one of the preceding claims, further comprising a weight adapted to be, in particular reversibly, attachable to the clip (4).

8. A method of determining of the shrinkage, the length and crimp properties of individual staple fibers by use of a testing apparatus according to one of claims 1 to 7, wherein
a) a staple fiber is fixed to an upper fixing board at its upper edge and to at least one clip at its lower, opposite edge, in particular without stretching the fiber,
b) said staple fiber is allowed to extend to its full length, in particular in front of a pad of contrasting color with regard to said fiber,
c) the length of said staple fiber is measured from the place where the fiber is fixed to the upper fixing board to the place where the lower edge of the fiber is fixed to the clip,
d) the fiber is relaxed by temporarily fixing the clip or the lower edge of the staple fiber onto or adjacent to the upper fixing board,
e) said fiber is exposed to a test environment, in particular to hot air and/or hot water,
f) and conditioned for a defined period of time after having been exposed to the test environment, and
g) the length of the shrunk fibers is/are measured and the result recorded,
**characterized in that**
a transparent or translucent upper fixing board and at least one transparent or translucent clip are used and that the upper fixing board, the at least one clip and the at least one staple fiber are illuminated at least when a property or properties of the staple fiber is/are determined during steps c) and g) and that the length of the staple fiber in steps c) and g) is measured by use of at least one lamp, a microscope and a digital monitor.

9. The method according to claim 8, **characterized in that**
in addition to the length measurement the shrinkage and crimp properties of the shrunk fiber is/are determined and the result recorded in step g).

10. The method according to claim 8 or 9, wherein
a grip weight is reversibly attached to the clip at least during the exposure to the testing environment.

11. The method according to one of claims 8 to 10, wherein
the testing environment comprises hot air, in particular from about 180° to about 195°C, or hot water, in particular from about 95° to about 99° C, in particular for about 15 minutes.

12. The method according to one of claims 8 to 11, wherein
the recorded data is transferred from the digital monitor to a computer.

13. The method according to one of claims 8 to 12, wherein
the length measurements on fibers are made in standard atmosphere, in particular at 20° to 22°C and at 63 to 67 % relative humidity.

14. A use of a testing apparatus according to one of claims 1 to 7 for measurement of the shrinkage, length and crimp properties of individual staple fibers.

## Patentansprüche

1. Prüfgerät (1) zur Bestimmung der Schrumpfung, Länge und Kräuseleigenschaften einzelner Stapelfasern, das eine obere Befestigungstafel (2) umfaßt, geeignet zur Befestigung von mindestens einem oberen Ende mindestens einer Stapelfaser (100), mindestens eine Klemme (4), geeignet zur wiederabnehmbaren Befestigung an einem unteren, entgegengesetzten Ende der Stapelfaser (100), **dadurch gekennzeichnet, daß** die obere Befestigungstafel (2) und die mindestens eine Klemme (4) durchsichtig oder transluzent sind und daß das Prüfgerät
mindestens eine Lampe (8) umfaßt, geeignet, um die obere Befestigungstafel (2), die Klemme oder Klemmen (4) und die Stapelfasern (100), wenn an der oberen Befestigungstafel (2) angebracht, zu beleuchten,
mindestens ein Mikroskop (12) und mindestens einen Digitalmonitor (10), beide gemeinsam dazu geeignet, die Faserlänge zu messen, aufzuzeichnen und die aufgezeichneten Daten an einen Mikroprozessor oder Computer (14) zu übertragen.

2. Das Prüfgerät (1) nach Anspruch 1, das ferner mindestens ein Heizgerät (6) umfaßt, um die Schrumpfung der Stapelfasern auszulösen.

3. Das Prüfgerät (1) nach Anspruch 1 oder 2, das ferner eine Unterlage (16), insbesondere aus Samt, in einer zu der Stapelfaser (100) kontrastierenden Farbe umfaßt, insbesondere mit schwarzer Oberfläche, welche sich hinter der Stapelfaser befindet.

4. Das Prüfgerät (1) nach einem der vorangehenden Ansprüche, das ferner ein Befestigungsmittel an oder bei der oberen Befestigungstafel (2) umfaßt, geeignet um die Klemme (4) und/oder das untere Ende der Stapelfaser (100) vorübergehend zu halten.

5. Das Prüfgerät (1) nach einem der vorangehenden Ansprüche, wobei die Klemme (4) und/oder die obere Befestigungstafel (2) aus durchsichtigem oder transluzentem Polymermaterial geformt ist/sind, insbesondere aus Polymethylmethacrylat (PMMA) und/oder Polycarbonat (PC) oder aus durchsichtigen oder transluzenten Gemischen, die Polymethylmethacrylat und/oder Polycarbonat enthalten.

6. Das Prüfgerät (1) nach einem der vorangehenden Ansprüche, wobei die obere Befestigungstafel (2) dazu geeignet ist, zumindest das obere Ende der zu prüfenden Stapelfaser (100) magnetisch einzuspannen oder zu halten.

7. Das Prüfgerät (1) nach einem der vorangehenden Ansprüche, das ferner ein Gewicht umfaßt, geeignet, um, insbesondere reversibel, an der Klemme (4) angebracht zu werden.

8. Verfahren zur Bestimmung der Schrumpfung, Länge und Kräuseleigenschaften einzelner Stapelfasern durch Verwendung des Prüfgeräts nach einem der Ansprüche 1 bis 7, wobei
a) eine Stapelfaser an ihrem oberen Ende an einer oberen Befestigungstafel und an ihrem unteren, entgegengesetzten Ende an mindestens einer Klemme befestigt wird, insbesondere ohne die Faser zu dehnen,
b) sich die Stapelfaser zu ihrer vollen Länge ausdehnen kann, insbesondere vor einer Unterlage von zu der Faser kontrastierender Farbe,
c) die Länge der Stapelfaser gemessen wird von der Stelle, an der die Faser an der oberen Befestigungstafel befestigt ist, bis zur Stelle, an der das untere Ende der Faser an der Klemme befestigt ist,
d) die Faser entspannt wird, indem die Klemme oder das untere Ende der Stapelfaser vorübergehend an oder bei der oberen Befestigungstafel befestigt wird.
e) die Faser einer Prüfumgebung, insbesondere heißer Luft und/oder heißem Wasser, ausgesetzt wird,
f) und, nachdem sie der Prüfumgebung ausgesetzt war, für eine festgelegte Zeitspanne klimatisiert wird, und
g) die Länge der geschrumpften Faser/n gemessen und aufgezeichnet wird/werden, **dadurch gekennzeichnet, daß**
eine durchsichtige oder transluzente obere Befestigungstafel und mindestens eine durchsichtige oder transluzente Klemme benutzt werden und daß die obere Befestigungstafel, die mindestens eine Klemme und die mindestens eine Stapelfaser beleuchtet werden, zumindest solange in den Schritten c) und g) eine Eigenschaft/Eigenschaften der Stapelfaser geprüft wird/werden und die Länge der Stapelfaser in den Schritten c) und g) mit Hilfe mindestens einer Lampe, eines Mikroskops und eines Digitalmonitors gemessen werden.

9. Das Verfahren nach Anspruch 8, **dadurch gekennzeichnet,**
**daß** zusätzlich zu der Längenmessung auch die Schrumpfung und Kräuseleigenschaften der geschrumpften Faser festgestellt wird/werden und das Ergebnis in Schritt g) aufgezeichnet wird.

10. Das Verfahren nach den Ansprüchen 8 und 9, wobei
zumindest während der Einwirkung der Prüfumgebung ein abnehmbares Klemmgewicht an der Klemme angebracht wird.

11. Das Verfahren nach einem der Ansprüche 8 bis 10, wobei
die Prüfumgebung vornehmlich circa 15 Minuten lang heiße Luft, insbesondere zwischen circa 180° bis 195°C, und heißes Wasser, insbesondere zwischen circa 95° bis 99°C, umfaßt.

12. Das Verfahren nach einem der Ansprüche 8 bis 11, wobei
die aufgezeichneten Daten vom Digitalmonitor auf den Computer übertragen werden.

13. Das Verfahren nach einem der Ansprüche 8 bis 12, wobei
die Längenmessungen an Fasern bei normaler Atmosphäre durchgeführt werden, vornehmlich bei 20° bis 22°C und einer relativen Luftfeuchtigkeit von 63 bis 67 %.

14. Verwendung des Prüfgeräts nach einem der Ansprüche 1 bis 7 zur Messung von Schrumpfung, Länge und Kräuseleigenschaften einzelner Stapelfasern.

## Revendications

1. Appareil de test (1) pour la détermination des propriétés de rétraction de longueur et de frisure des fibres discontinues individuelles comprenant une carte de fixation supérieure (2) adaptée pour maintenir au moins un bord supérieur d'au moins une fibre discontinue (100), au moins un clip (4) adapté pour être placé de manière réversible sur un bord opposé inférieur de la fibre discontinue (100), **caractérisé en ce que** la carte de fixation supérieure (2) et le au moins un clip (4) sont transparents ou translucides et **en ce que** l'appareil de test comprend
au moins une lampe (8) adaptée pour éclairer la carte de fixation supérieure (2), le clip ou les clips (4) et les fibres discontinues (100) lorsque fixée à la carte de fixation supérieure (2),
au moins un microscope (12) et au moins un écran numérique (10), les deux en combinaison étant adaptés pour mesurer et pour enregistrer la longueur de fibre et pour transférer les données enregistrées sur un microprocesseur ou un ordinateur (14).

2. Appareil de test (1) selon la revendication (1), comprenant en outre au moins un dispositif de chauffage (6) pour initier la rétraction des fibres discontinues.

3. Appareil de test (1) selon la revendication 1 ou 2, comprenant, en outre, un tampon (16), dans un velours particulier, d'une couleur contrastée par rapport à la fibre discontinue (100) et étant derrière la fibre discontinue, en particulier ayant une surface noire.

4. Appareil de test (1) selon l'une quelconque des revendications précédentes, comprenant, en outre, un moyen de fixation sur ou adjacent à la carte de fixation (2) supérieure adaptée pour maintenir temporairement le clip (4) et/ou le bord inférieur de la fibre discontinue (5).

5. Appareil de test (1) selon l'une quelconque des revendications précédentes, dans lequel le clip (4) et/ou la carte de fixation (2) est/sont constitué(s) d'un matériau polymère transparent ou translucide, en particulier de polyméthylméthacrylate (PMMA) et/ou de polycarbonate (PC) ou des mélanges transparents ou translucides comprenant du polyméthylméthacrylate et/ou du polycarbonate.

6. Appareil de test (1) selon l'une quelconque des revendications précédentes, dans lequel la carte de fixation supérieure (2) est adaptée pour serrer ou maintenir magnétiquement au moins le bord supérieur de la fibre discontinue (100) qui doit être testée.

7. Appareil de test (1) selon l'une quelconque des revendications précédentes, comprenant, en outre, un poids adapté pour être, en particulier de manière réversible, attachable au clip (4).

8. Procédé de détermination des propriétés de rétraction de longueur et de frisure des fibres discontinues individuelles par l'utilisation d'un appareil de test selon l'une quelconque des revendications 1 à 7, dans lequel
a) une fibre discontinue est fixée à une carte de fixation supérieure au niveau de son bord supérieur et à au moins un clip au niveau de son bord inférieur opposé, en particulier, sans étirer la fibre,
b) ladite fibre discontinue est autorisée à s'étendre sur sa longueur entière, en particulier à l'avant d'un tampon d'une couleur contrastée eu égard à ladite fibre,
c) la longueur de ladite fibre discontinue est mesurée à partir de l'emplacement où la fibre est fixée à la carte de fixation supérieure à l'emplacement où le bord inférieur de la fibre est fixé au clip,
d) la fibre est relâchée en fixant temporairement le clip ou le bord inférieur de la fibre discontinue sur ou adjacent à la carte de fixation supérieure,
e) ladite fibre est exposée à un environnement de test, en particulier à de l'air chaud et/ou de l'eau chaude,
f) et à la conditionner pendant une période de temps définie après avoir été exposée à l'environnement de test, et
g) la longueur de la fibre ou des fibres rétractée(s) est/sont mesurées et le résultat enregistré,
**caractérisé en ce que**
une carte de fixation supérieure transparente ou translucide et au moins un clip transparent ou translucide sont utilisés et **en ce que** la carte de fixation supérieure, le au moins un clip et la au moins une fibre discontinue sont éclairés au moins lorsqu'une propriété ou des propriétés de la fibre discontinue est/sont déterminée(s) pendant les étapes c) et g) et **en ce que** la longueur de la fibre discontinue aux étapes c) et g) est mesurée par l'utilisation d'au moins une lampe, un microscope et un écran numérique.

9. Procédé selon la revendication 8, **caractérisé en ce que**
en plus de la mesure de la longueur, les propriétés de rétraction et de frisure de la fibre rétractée est/sont déterminées et le résultat est enregistré à l'étape g).

10. Procédé selon la revendication 8 ou 9, dans lequel
un poids de saisie est fixé de manière réversible au clip au moins pendant l'exposition à l'environnement de test.

11. Procédé selon l'une quelconque des revendications 8 à 10, dans lequel
l'environnement de test comprend de l'air chaud, en particulier à une température d'environ 180° à environ 195°C, ou de l'eau chaude, en particulier à une température d'environ 95° à environ 99°C, en particulier pendant environ 15 minutes.

12. Procédé selon l'une quelconque des revendications 8 à 11, dans lequel
les données enregistrées sont transférées de l'écran numérique à un ordinateur.

13. Procédé selon l'une quelconque des revendications 8 à 12, dans lequel
les mesures de longueur sur les fibres sont effectuées dans une atmosphère standard, en particulier à des températures de 20° à 22°C et à une humidité relative de 63 à 67 %.

14. Utilisation d'un appareil de test selon l'une quelconque des revendications 1 à 7 pour la mesure des propriétés de rétraction de longueur et de frisure des fibres discontinues individuelles.
